# FASCICULE DE BREVET EUROPEEN

(11) **EP 2 411 588 B1**
(45) Date de publication et mention de la délivrance du brevet: **11.10.2017**
(21) Numéro de dépôt: 10715957.6
(22) Date de dépôt: 19.03.2010
(51) Int. Cl.: E03B 7/00, C02F 1/00, G01N 33/18

(54) **INSTALLATION ET PROCEDE DE CONTROLE DE LA QUALITE DE L'EAU DANS UN RESEAU D'EAU POTABLE**
EINRICHTUNG UND VERFAHREN ZUR ÜBERWACHUNG DER WASSERQUALITÄT IN EINEM TRINKWASSERNETZ
EQUIPMENT AND METHOD FOR MONITORING THE QUALITY OF WATER IN A DRINKING WATER NETWORK

(30) Priorité: 24.03.2009 FR 0951859
(43) Date de publication de la demande: 01.02.2012
(73) Titulaire: Veolia Eau - Compagnie Generale Des Eaux, 75008 Paris (FR)
(72) Inventeur: LEMOINE, Cyrille, F-78500 Sartrouville (FR); MOREAU, Marc, F-78220 Viroflay (FR)
(74) Mandataire: Cardy, Sophie Marie
(86) Numéro de dépôt international: PCT/FR2010/050497
(87) Numéro de publication internationale: WO 2010/109117

(56) Documents cités:
- FR-A- 2 911 960
- FR-A- 2 915 755
- US-B1- 6 245 224

## Description

La présente invention concerne le domaine du contrôle de la qualité de l'eau dans les réseaux de distribution d'eau potable.

Actuellement, bien que la qualité de l'eau dans les réseaux soit globalement maîtrisée, des contaminations ou pollutions ponctuelles peuvent néanmoins apparaître à la suite de ruptures ou casses de conduites, ou en raison de retours d'eau, ou bien encore à cause d'intrusions volontaires de contaminants dans le réseau d'eau potable.

Par retour d'eau, on entend un flux d'eau s'écoulant de manière anormale depuis le consommateur vers le réseau d'eau potable.

Certains grands consommateurs d'eau, comme par exemple les hôpitaux, les crèches, les maisons de retraite, les restaurateurs et plus généralement les établissements accueillant du public, sont particulièrement sensibles au risque de contamination.

Pour contrôler la qualité de l'eau dans le réseau, le document WO 2007/011352 propose de filtrer et purifier l'eau au point de livraison (à proximité du consommateur) à l'aide de cartouches de filtration et purification.

L'inconvénient de ce système est qu'il nécessite de changer régulièrement les cartouches, ce qui se révèle onéreux et malcommode. De plus, en cas de contamination sévère, l'utilisation d'une cartouche filtrante peut se révéler insuffisante pour bloquer la contamination.

Le document US 6 245 224 décrit une installation de contrôle de la qualité de l'eau mais ne s'intéresse pas aux retours d'eau. En outre, cette installation est très complexe dans la mesure où elle nécessite une pluralité de capteurs de qualité dans le réseau, des capteurs de débit, un système de communication bidirectionnelle ainsi qu'un système central d'analyse des données.

Un but de la présente invention est de proposer une installation de contrôle de la qualité de l'eau, destinée à être montée sur une conduite dédiée à la distribution d'eau à un consommateur, qui vise à pallier les inconvénients précités.

L'invention atteint son but par le fait que l'installation comprend les caractéristiques de la revendication 1. C'est ainsi que grâce à l'invention, le dispositif de désinfection n'est activé que lorsque cela est nécessaire, autrement dit lorsqu'une contamination est détectée. L'énergie nécessaire pour faire fonctionner le dispositif de désinfection est ainsi minimisée.

En outre, en cas de contamination sévère, la vanne de sectionnement est actionnée de manière à obturer la conduite de manière à protéger le consommateur, si la contamination provient du réseau de distribution, ou le réseau si la contamination provient du consommateur.

De plus, la gestion des retours d'eau est améliorée par rapport à l'art antérieur dans la mesure où les moyens de sécurisation à savoir le dispositif de désinfection et/ou la vanne de sectionnement sont préférentiellement actionnés uniquement lorsqu'un retour d'eau est détecté par le dispositif de détection de flux inverse en même temps qu'une contamination.

Un autre avantage de cette installation est son autonomie. En outre, contrairement à l'art antérieur, l'installation selon l'invention permet une sécurisation locale du consommateur dès lors que l'ensemble de l'installation est montée sur une conduite qui relie le consommateur au reste du réseau.

De préférence, l'installation comporte en outre un compteur de consommation comprenant ledit dispositif de détection de flux inverse.

Le compteur de consommation est l'équipement qui mesure le volume d'eau consommé par le consommateur. Les compteurs sont périodiquement relevés par un opérateur ou automatiquement par télérelevé.

Avantageusement, l'installation comporte en outre un dispositif d'alerte du consommateur qui est destiné à être activé en fonction des données fournies par le dispositif de détection de contamination et le dispositif de détection de flux inverse.

Ainsi, en cas de détection d'une contamination, ou bien en cas de détection d'un retour d'eau associé à la détection d'une contamination, une alerte est déclenchée afin d'alerter le consommateur.

Selon un mode de réalisation avantageux, l'installation comporte en outre une unité de transmission pour transmettre à un centre de contrôle du réseau de distribution les informations recueillies par le dispositif de détection de contamination et le dispositif de détection de flux inverse, ainsi que l'état du dispositif de désinfection et/ou l'état de la vanne.

Le centre de contrôle recueille les informations provenant de l'ensemble des installations du réseau d'eau potable. Les alertes sont donc également transmises au centre de contrôle. La transmission est de préférence réalisée par des moyens de transmission sans fil.

Le cas échéant, le centre de contrôle est à même d'établir une cartographie de la contamination et éventuellement fermer une ou plusieurs canalisations du réseau afin d'assurer la sécurité des consommateurs.

De préférence, ledit paramètre mesuré par la sonde est pris au moins parmi la concentration en chlore, la conductivité, la température, la turbidité et la concentration en matière organique.

Encore de préférence, la sonde est du type multicapteurs en ce sens qu'elle mesure une pluralité de paramètres.

En outre, la sonde réalise avantageusement les mesures sans utiliser de réactif ni rejet d'eau à l'extérieur de la conduite.

En d'autres termes, les mesures sont effectuées sans gaspillage d'eau.

Avantageusement, le dispositif de désinfection selon l'invention, contrairement à l'art antérieur, n'utilise ni consommables (telles les cartouches filtrantes) ni réactifs, grâce à quoi l'installation selon l'invention présente un coût d'exploitation et une empreinte environnementale réduits par rapport à ceux de l'art antérieur.

Avantageusement, le dispositif de désinfection comprend un système électrochimique générant des composés oxydants, par exemple du type chlore sous ses différentes formes, ou encore des peroxydes, percarbonates, ou bien de l'ozone.

Les composés oxydants sont donc générés in situ ce qui permet d'éviter le stockage de produits chimiques chez le consommateur.

Avantageusement, le dispositif de désinfection comprend une lampe ultra-violet, qui peut être associée, ou non, au système électrochimique précité.

Selon un aspect particulièrement avantageux de l'invention, la sonde mesure au moins deux paramètres, et le dispositif de détection de contamination comprend des moyens pour réaliser une analyse corrélée desdits au moins deux paramètres.

Le principe de l'analyse corrélée de plusieurs paramètres est expliqué dans le document FR 2 911 960. Il est basé sur l'analyse croisée d'un ensemble d'indicateurs non pertinents à eux seuls mais dont le rapprochement permet de reconnaître un problème de qualité justifiant une mesure de sauvegarde sans avoir identifié exactement sa nature. Grâce à cet algorithme, il est possible de distinguer la variabilité naturelle de la variabilité accidentelle des paramètres mesurés, et par voie de conséquence, d'éviter les fausses alertes. Bien évidement, sans sortir du cadre de la présente invention, le système de détection de contamination peut être basé sur tout autre algorithme, par exemple du type à seuil de déclenchement.

L'invention concerne en outre une conduite de branchement, dédiée à la distribution d'eau à un consommateur depuis une conduite de distribution reliée à une conduite de transfert acheminant de l'eau depuis un réservoir, sur laquelle est montée une installation selon l'invention.

L'invention concerne en outre un réseau de distribution comportant une pluralité de conduites de distribution reliées à au moins une conduite de transfert acheminant de l'eau depuis un réservoir, une pluralité de conduites de branchement reliées aux conduites de distribution pour la distribution d'eau à des consommateurs, et au moins une installation selon l'invention, ladite installation étant montée sur l'une desdites conduites de branchement.

Selon l'invention, l'installation est montée sur la conduite de branchement, c'est-à-dire la conduite qui relie le point de livraison (le consommateur) à une canalisation principale du réseau.

Avantageusement, le réseau de distribution selon l'invention comporte en outre un centre de contrôle recevant les informations fournies par le dispositif de détection de contamination et le dispositif de détection de flux inverse, ainsi que l'état du dispositif de désinfection et/ou l'état de la vanne, le centre de contrôle comportant en outre des moyens pour piloter à distance l'organe de commande de l'installation.

Ainsi, en cas de détection d'une contamination chez un ou plusieurs consommateurs, le centre de contrôle est apte à actionner la vanne de sectionnement et/ou activer le dispositif de désinfection de toute installation de contrôle du réseau. Par suite, en cas de détection d'une contamination chez un consommateur, l'opérateur du centre de contrôle pourra par exemple activer les moyens de sécurisation des consommateurs voisins de celui où la contamination a été détectée.

De manière avantageuse, le centre de contrôle comporte en outre un modèle hydraulique et/ou un modèle qualité recevant les données fournies par le dispositif de détection de contamination, de manière à prédire la propagation de la contamination éventuellement détectée.

Ainsi, la présente invention permet une supervision globale du réseau de distribution. La prédiction de la diffusion de la contamination permet de prendre rapidement des mesures ciblées en vue de préserver la sécurité des consommateurs.

L'invention concerne enfin un procédé de contrôle de la qualité de l'eau dans un réseau de distribution, comportant au moins une installation de contrôle selon l'invention, procédé dans lequel on actionne le dispositif de désinfection et/ou la vanne de sectionnement lorsqu'un flux inverse est détecté et/ou lorsqu'une contamination est détectée.

Comme on l'a déjà indiqué ci-dessus, la vanne de sectionnement est préférentiellement mais pas exclusivement actionnée lorsqu'une contamination et un retour d'eau sont simultanément détectés.

De préférence, une alerte est en outre déclenchée si le dispositif de désinfection et/ou la vanne de sectionnement sont actionnés.

L'invention sera mieux comprise et ses avantages apparaîtront mieux à la lecture de la description détaillée qui suit, d'un mode de réalisation indiqué à titre d'exemple non limitatif. La description se réfère aux figures annexées suivantes :
- la figure **1** illustre une partie d'un réseau de distribution d'eau potable comprenant une pluralité d'installations de contrôle selon l'invention et
- la figure **2** montre un mode de réalisation de l'installation de contrôle selon l'invention.

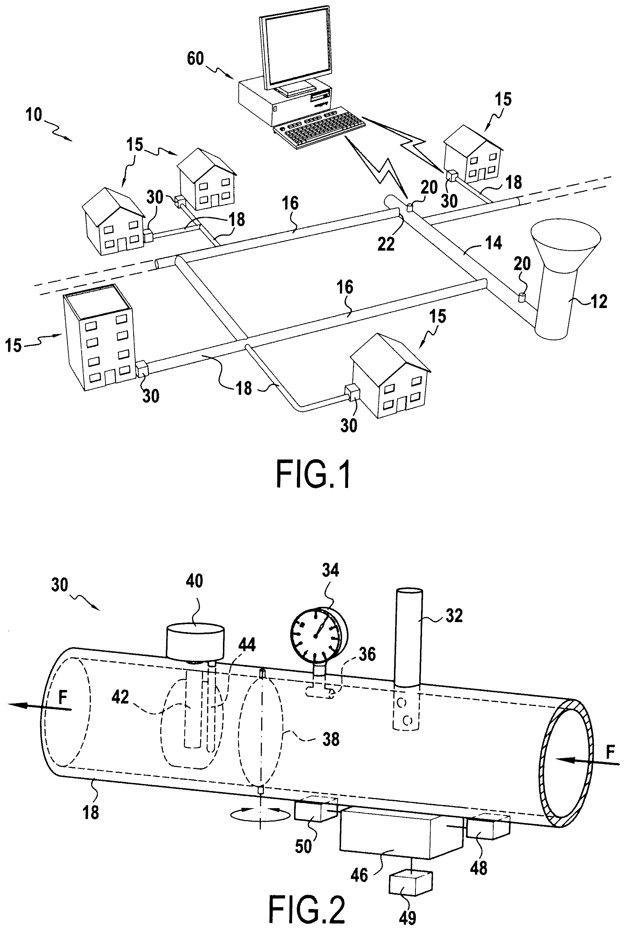

Le réseau de distribution d'eau potable **10** représenté sur la figure **1** correspond à la partie aval d'un réseau principal alimenté par une ou plusieurs usines de production d'eau (non représentées ici).

Ce réseau **10** est alimenté par un réservoir **12,** en l'espèce un château d'eau, lui-même relié au réseau principal par des canalisations non représentées ici.

Classiquement, l'eau potable est acheminée depuis le réservoir **12** par des conduites de transfert **14** dédiées à l'acheminement d'eau sur une longue distance. Une pluralité de conduites de distribution **16** sont reliées aux conduites de transfert **14,** et chaque consommateur **15,** ou abonné, est quant à lui relié aux conduites de distribution **16** par l'intermédiaire de conduites de branchement **18.** Par consommateur, on entend une personne individuelle ou un groupe de personnes consommant de l'eau, comme par exemple un hôpital, une crèche, un immeuble ou plusieurs maisons individuelles reliées à une même conduite de branchement. On comprend donc que dans la plupart des cas le diamètre des conduites de branchement **18** est sensiblement inférieur aux diamètres des conduites de transfert **14** et distribution **16.**

Le réseau **10** est en outre préférentiellement équipé de capteurs **20,** à savoir des capteurs hydrauliques du type capteurs de pression ou capteurs de débit, ainsi que des capteurs pour la mesure de concentration de certaines espèces chimiques comme le chlore par exemple.

Dans l'exemple de la figure **1****,** les capteurs **20** sont montés sur l'un des noeuds **22** du réseau **10,** et en sortie immédiate du réservoir **12.** Bien évidemment, le nombre de capteurs illustrés n'est pas limitatif et l'on peut prévoir davantage de capteurs ainsi que des localisations différentes.

Conformément à l'invention, le réseau de distribution **10** comporte une pluralité d'installations de contrôle **30** qui sont en l'espèce montées sur les conduites de branchement **18.**

Selon l'invention, l'installation de contrôle **30,** mieux visible sur la figure **2****,** comporte une sonde **32** du type multicapteurs qui est installé sur la conduite de branchement, en amont de celle-ci par rapport au sens de circulation normal **F** de l'eau dans la conduite **18.**

Cette sonde **32** réalise les mesures sans rejet d'eau à l'extérieur de la conduite **18** et ne nécessite pas l'utilisation de réactifs.

Qui plus est, la sonde **32** est apte à mesurer plusieurs paramètres, à savoir la concentration en chlore, la conductivité, la température de l'eau, la turbidité, ainsi que la concentration en matière organique. Cette sonde **32** est en l'espèce préférentiellement composée de plusieurs capteurs.

L'installation **30** comprend en outre, en aval de la sonde **32,** un compteur de consommation **34** qui est équipé d'un moyen de télérelevé, connu par ailleurs. Le compteur de consommation **34** comporte par ailleurs un dispositif de détection de flux inverse **36.** Par flux inverse, on entend un flux d'eau s'écoulant dans le sens opposé au sens normal **F,** c'est-à-dire du réseau vers le consommateur.

L'installation **30** comporte aussi une vanne de sectionnement **38** qui, lorsqu'elle est actionnée, permet d'obturer la conduite de branchement **18.**

De plus, l'installation **30** comporte un dispositif de désinfection **40** disposé en aval du compteur **34,** de la vanne de sectionnement **38** et de la sonde **32.** Ce dispositif de désinfection **40** comprend un système électrochimique **42** générant in situ des composés oxydants dans la conduite **18,** ainsi que, de préférence, une lampe UV **44.** Les composés oxydants, dans cet exemple, sont le chlore sous différentes formes, des peroxydes, l'ozone et des radicaux hydroxyles. On comprend donc que, lorsqu'il est actionné, le dispositif de désinfection permet de désinfecter l'eau s'écoulant dans la conduite **18.**

L'installation **30** comporte en outre un dispositif de détection de contamination **46** auquel est reliée la sonde **32** par des moyens de transmission sans-fil **48.**

Le dispositif de détection de contamination a pour fonction de détecter la présence éventuelle d'une contamination à partir des mesures effectuées par la sonde **32.** En l'espèce, le dispositif de détection met en oeuvre l'algorithme d'analyse corrélée qui est décrit dans le document FR 2 911 960.

Par exemple, la détection simultanée d'une baisse du taux de chlore et d'une hausse de la turbidité associé à un flux d'eau normal allant du réseau **10** vers le consommateur **15** signale une contamination probable de l'eau. Une telle contamination peut provenir d'une casse ou du décollement du biofilm recouvrant la paroi intérieur des conduites du réseau **10.**

Pour l'actionnement de la vanne de sectionnement **38** et du dispositif de désinfection **40,** l'installation de contrôle **30** comporte en outre un organe de commande **50** relié au dispositif de détection **46** d'une part, et à la vanne de sectionnement **38** ainsi qu'au dispositif de désinfection **40** d'autre part.

De préférence, l'organe de commande **50** est relié à la vanne de sectionnement ainsi qu'au dispositif de désinfection **40** par les moyens de transmission **48.**

L'organe de commande a donc pour fonction de commander le dispositif de détection de contamination **46** et la vanne de sectionnement **38,** ensemble ou de manière séparée.

Selon un premier mode de fonctionnement, l'organe de commande actionne le dispositif de désinfection **40** si une contamination est détectée par le dispositif de détection de contamination **46.** Si aucun retour d'eau n'est détecté par le dispositif de détection de flux inverse, alors la vanne de sectionnement ne sera généralement pas actionnée, sauf si la contamination détectée est particulièrement importante.

En revanche et selon un second mode de fonctionnement, si une contamination et un retour d'eau sont simultanément détectés, la vanne de sectionnement est actionnée de manière à obturer la conduite **18.** On évite ainsi la propagation du polluant dans le réseau. Dans ce second mode de fonctionnement, le dispositif de désinfection peut éventuellement être activé.

Par exemple, un retour d'eau associé à une hausse de la température signale une probable connexion du réseau d'alimentation en eau potable avec le réseau d'eau chaude sanitaire du consommateur. Il peut en résulter un risque de développement de légionnelles chez le consommateur, voire une contamination du réseau de distribution **10** si le retour d'eau est important. Selon l'invention, la gravité du retour d'eau est qualifiée en fonction du volume d'eau s'écoulant vers le réseau **10** et du type de contamination détectée.

L'installation de contrôle **30** comporte également des moyens d'alerte pour indiquer au consommateur **15** la présence d'une contamination.

Selon un aspect avantageux de l'invention, le réseau de distribution **10** comporte en outre un centre de contrôle **60** dont la fonction est de contrôler l'exploitation du réseau **10.** Pour ce faire, le centre de contrôle **60** comprend un système de traitement informatique permettant de modéliser le comportement hydraulique et qualité de l'eau dans le réseau. Le centre de contrôle **60** comporte donc un modèle hydraulique couplé à un modèle dit « qualité », par exemple un modèle cinétique de décroissance de la concentration en chlore. Il convient de préciser que de tels modèles sont déjà connus.

Le centre de contrôle recueille notamment les informations provenant des différents capteurs **20** ainsi que des installations de contrôle **30.**

Autrement dit, chaque installation de contrôle **30** comporte une unité de transmission **49** pour envoyer au centre de contrôle **60** les informations émanant du dispositif de détection de contamination **46** et du dispositif de détection de flux inverse **36.** Les unités de transmission **49** envoient également au centre de contrôle l'état de la vanne de sectionnement **38,** à savoir « ouvert » ou « fermé », ainsi que l'état du dispositif de désinfection **40,** à savoir « actif » ou « inactif ».

Ces informations, fournies aux modèles hydraulique et qualité, permettent de superviser le réseau de distribution de manière globale. En effet, les modèles hydraulique et qualité permettent avantageusement de simuler la propagation d'une contamination détectée en un point du réseau, et de déclencher le cas échéant des actions préventives. Pour ce faire, le centre de contrôle **60** comporte en outre des moyens pour piloter à distance les organes de commande **50** des différentes installations de contrôle du réseau **10.**

Ainsi, lorsqu'une contamination est détectée chez un ou plusieurs consommateurs **15,** le centre de contrôle **60** est apte à actionner les vannes de sectionnement **38** des installations de contrôle **30** chez les consommateurs **15** qui sont situés en aval de la propagation telle que prédite par les modèles précités. D'autres choix d'isolement de parties du réseau **10** peuvent également être envisagés.

Qui plus est, le centre de contrôle **60** du réseau **10** selon l'invention est apte à identifier les zones de contamination à partir des informations recueillies par les dispositifs de détection de contamination **46.**

Par ailleurs et de préférence, les données transmises au sein de l'installation de contrôle **30** d'une part, et entre l'installation **30** et le centre de contrôle **60** d'autre part, sont avantageusement cryptées.

## Revendications

1. Installation (18) de contrôle (30) de la qualité de l'eau destinée à être montée sur une conduite de branchement (18) dédiée à la distribution d'eau à un consommateur (15) depuis une conduite de distribution (16) reliée à un conduite de transfert (14) acheminant de l'eau depuis un réservoir (12), ladite installation étant **caractérisée en ce qu'**elle comprend :
- une sonde (32) pour mesurer au moins un paramètre de l'eau circulant dans la conduite de branchement (18) ;
- un dispositif de détection de contamination (46) pour détecter la présence éventuelle d'une contamination à partir des mesures effectuées par la sonde ;
- un dispositif de détection de flux inverse (36) dans ladite conduite de branchement (18) ;
- un dispositif de désinfection (40) pour désinfecter l'eau dans la conduite de branchement (18) ;
- une vanne de sectionnement (38) pour obturer la conduite de branchement (18) ; et
- un organe de commande (50) pour actionner le dispositif de désinfection (40) et/ou la vanne de sectionnement (38) en fonction des données fournies par le dispositif de détection de contamination (46) et le dispositif de détection de flux inverse (36), par quoi on contrôle la qualité de l'eau distribuée au ou refoulée par le consommateur (15),
l'ensemble de l'installation étant configuré pour être monté sur ladite conduite de branchement (18) reliant le consommateur au reste du réseau.

2. Installation selon la revendication **1, caractérisée en ce qu'**elle comporte en outre un compteur de consommation (34) comprenant ledit dispositif de détection de flux inverse (36).

3. Installation selon la revendication **1** ou **2, caractérisée en ce qu'**elle comporte en outre un dispositif d'alerte du consommateur (15) destiné à être activé en fonction des données fournies par le dispositif de détection de contamination (46) et le dispositif de détection de flux inverse (36).

4. Installation selon l'une quelconque des revendications **1** à **3, caractérisée en ce qu'**elle comporte en outre une unité de transmission (49) pour transmettre à un centre de contrôle (60) du réseau de distribution (10) les informations recueillies par le dispositif de détection de contamination (46) et le dispositif de détection de flux inverse (36), ainsi que l'état du dispositif de désinfection (40) et/ou l'état de la vanne de sectionnement (38).

5. Installation selon l'une quelconque des revendications **1** à **4, caractérisée en ce que** ledit paramètre est pris au moins parmi la concentration en chlore, la conductivité, la température, la turbidité et la concentration en matière organique.

6. Installation selon l'une quelconque des revendications **1** à **5, caractérisée en ce que** le dispositif de désinfection (40) comprend un système électrochimique (42) générant des composés oxydants.

7. Installation selon l'une quelconque des revendications **1** à **6, caractérisée en ce que** le dispositif de désinfection comprend une lampe ultra-violet (44).

8. Installation selon l'une quelconque des revendications **1** à **7, caractérisée en ce que** la sonde mesure au moins deux paramètres, et **en ce que** le dispositif de détection de contamination (46) comprend des moyens pour réaliser une analyse corrélée desdits au moins deux paramètres.

9. Installation selon l'une quelconque des revendications **1** à **8, caractérisée en ce que** la sonde (32) réalise les mesures sans utiliser de réactif ni rejet d'eau à l'extérieur de la conduite de branchement (18).

10. Conduite de branchement, dédiée à la distribution d'eau à un consommateur (15) depuis une conduite de distribution (16) reliée à un conduite de transfert (14) acheminant de l'eau depuis un réservoir (12), sur laquelle est monté une installation (30) selon l'une quelconque des revendications **1** à **9.**

11. Réseau de distribution (10) comportant une pluralité de conduites de distribution (16) reliées à au moins une conduite de transfert (14) acheminant de l'eau depuis un réservoir (12), une pluralité de conduites de branchement (18) reliées aux conduites de distribution (16) pour la distribution d'eau à des consommateurs, et au moins une installation (30) selon l'une quelconque des revendications **1** à **9,** ladite installation étant montée sur l'une desdites conduites de branchement (18).

12. Réseau de distribution selon la revendication **11, caractérisé en ce qu'**il comporte en outre un centre de contrôle (60) recevant les informations fournies par le dispositif de détection de contamination (46) et le dispositif de détection de flux inverse (36), ainsi que l'état du dispositif de désinfection et/ou l'état de la vanne de sectionnement, et **en ce que** le centre de contrôle comporte en outre des moyens pour piloter à distance l'organe de commande de l'installation.

13. Réseau de distribution selon la revendication **12, caractérisé en ce que** le centre de contrôle (60) comporte en outre un modèle hydraulique et/ou un modèle qualité recevant les données fournies par le dispositif de détection de contamination (46), de manière à prédire la propagation dans le réseau de la contamination éventuellement détectée.

14. Procédé de contrôle de la qualité de l'eau dans un réseau de distribution, comportant au moins une installation selon l'une quelconque des revendications **1** à **9,** procédé dans lequel on actionne le dispositif de désinfection (40) et/ou la vanne de sectionnement (38) lorsqu'un flux inverse est détecté et/ou lorsqu'une contamination est détectée.

15. Procédé de contrôle de la qualité de l'eau selon la revendication **14,** dans lequel une alerte est déclenchée si le dispositif de désinfection (40) et/ou la vanne de sectionnement (38) sont actionnés.

## Patentansprüche

1. Einrichtung (18) zur Überwachung (30) der Wasserqualität, die dazu bestimmt ist, auf einer Zweigleitung (18) montiert zu sein, die für die Verteilung von Wasser an einen Verbraucher (15) von einer Verteilungsleitung (16) bestimmt ist, die mit einer Transferleitung (14) verbunden ist, die Wasser von einem Reservoir (12) zuleitet, wobei die Einrichtung umfasst:
- eine Sonde (32) zum Messen mindestens eines Parameters des in der Zweigleitung (18) zirkulierenden Wassers,
- eine Vorrichtung zur Erfassung einer Kontamination (46), um das mögliche Vorhandensein einer Kontamination auf Basis von von der Sonde durchgeführten Messungen zu erfassen,
- eine Vorrichtung zur Erfassung eines Rückflusses (36) in die Zweigleitung (18),
- eine Desinfektionsvorrichtung (40), um das Wasser in der Zweigleitung (18) zu desinfizieren,
- ein Absperrventil (38), um die Zweigleitung (18) zu verschließen, und
- ein Steuerelement (50), um die Desinfektionsvorrichtung (40) und/oder das Absperrventil (38) in Abhängigkeit von Daten, die von der Vorrichtung zur Erfassung einer Kontamination (46) und der Vorrichtung zur Erfassung eines Rückflusses (36) geliefert werden, zu betätigen, wodurch die Qualität des an den Verbraucher (15) verteilten oder von diesem kommenden Wassers überwacht wird,
wobei die gesamte Einrichtung dazu vorgesehen ist, auf der Zweigleitung (18), die den Verbraucher mit dem übrigen Netz verbindet, montiert zu sein.

2. Einrichtung gemäß Anspruch 1, **dadurch gekennzeichnet, dass** sie ferner einen Verbrauchszähler (34) umfasst, der die Vorrichtung zur Erfassung eines Rückflusses (36) umfasst.

3. Einrichtung gemäß Anspruch 1 oder 2, **dadurch gekennzeichnet, dass** sie ferner eine Vorrichtung zur Warnung des Verbrauchers (15) umfasst, die dazu bestimmt ist, in Abhängigkeit von den von der Vorrichtung zur Erfassung einer Kontamination (46) und der Vorrichtung zur Erfassung eines Rückflusses (36) gelieferten Daten aktiviert zu werden.

4. Einrichtung gemäß einem der Ansprüche 1 bis 3, **dadurch gekennzeichnet, dass** sie ferner eine Übertragungseinheit (49) umfasst, um an ein Überwachungszentrum (60) des Verteilungsnetzes (10) die von der Vorrichtung zur Erfassung einer Kontamination (46) und der Vorrichtung zur Erfassung eines Rückflusses (36) gesammelten Informationen sowie den Zustand der Desinfektionsvorrichtung (40) und/oder den Zustand des Absperrventils (38) zu übertragen.

5. Einrichtung gemäß einem d er Ansprüche 1 bis 4, **dadurch gekennzeichnet, dass** der Parameter mindestens unter der Chlorkonzentration, der Leitfähigkeit, der Temperatur, der Trübung und der Konzentration an organischen Stoffen gewählt ist.

6. Einrichtung gemäß einem der Ansprüche 1 bis 5, **dadurch gekennzeichnet, dass** die Desinfektionsvorrichtung (40) ein elektrochemisches System (42) umfasst, das oxidierende Verbindungen erzeugt.

7. Einrichtung gemäß einem der Ansprüche 1 bis 6, **dadurch gekennzeichnet, dass** die Desinfektionsvorrichtung eine UV-Lampe (44) umfasst.

8. Einrichtung gemäß einem der Ansprüche 1 bis 7, **dadurch gekennzeichnet, dass** die Sonde mindestens zwei Parameter misst, und dass die Vorrichtung zur Erfassung einer Kontamination (46) Mittel umfasst, um eine korrelierte Analyse der mindestens zwei Parameter durchzuführen.

9. Einrichtung gemäß einem der Ansprüche 1 bis 8, **dadurch gekennzeichnet, dass** die Sonde (32) die Messungen durchführt, ohne ein Reagens oder ein Ablassen von Wasser aus der Zweigleitung (18) zu verwenden.

10. Zweigleitung, die für die Verteilung von Wasser an einen Verbraucher (15) von einer Verteilungsleitung (16), die mit einer Transferleitung (14) verbunden ist, die Wasser von einem Reservoir (12) zuleitet, bestimmt ist, auf der eine Einrichtung (30) gemäß einem der Ansprüche 1 bis 9 montiert ist.

11. Verteilungsnetz (10), umfassend eine Vielzahl von Verteilungsleitungen (16), die mit mindestens einer Transferleitung (14) verbunden sind, die Wasser von einem Reservoir (12) zuleitet, eine Vielzahl von Zweigleitungen (18), die mit den Verteilungsleitungen (16) zur Verteilung von Wasser an Verbraucher verbunden sind, und mindestens eine Einrichtung (30) gemäß einem der Ansprüche 1 bis 9, wobei die Einrichtung auf einer der Zweigleitungen (18) montiert ist.

12. Verteilungsnetz gemäß Anspruch 11, **dadurch gekennzeichnet, dass** es ferner ein Überwachungszentrum (60) umfasst, das die von der Vorrichtung zur Erfassung einer Kontamination (46) und der Vorrichtung zur Erfassung eines Rückflusses (36) gelieferten Informationen sowie den Zustand der Desinfektionsvorrichtung und/oder den Zustand des Absperrventils empfängt, und dass das Überwachungszentrum ferner Mittel umfasst, um das Steuerelement der Einrichtung fernzusteuern.

13. Verteilungsnetz gemäß Anspruch 12, **dadurch gekennzeichnet, dass** das Überwachungszentrum (60) ferner ein Hydraulikmodell und/oder ein Qualitätsmodell umfasst, das die von der Vorrichtung zur Erfassung einer Kontamination (46) gelieferten Daten empfängt, um die Ausbreitung der eventuell erfassten Kontamination in dem Netz vorherzusagen.

14. Verfahren zur Überwachung der Wasserqualität in einem Verteilungsnetz, umfassend mindestens eine Einrichtung gemäß einem der Ansprüche 1 bis 9, wobei bei dem Verfahren die Desinfektionsvorrichtung (40) und/oder das Absperrventil (38) bestätigt werden, wenn ein Rückfluss erfasst und/oder wenn eine Kontamination erfasst wird.

15. Verfahren zur Überwachung der Wasserqualität gemäß Anspruch 14, bei dem ein Alarm ausgelöst wird, wenn die Desinfektionsvorrichtung (40) und/oder das Absperrventil (38) betätigt werden.

## Claims

1. A monitoring installation (18, 30) for monitoring water quality and intended to be mounted on a connection pipe (18) dedicated to delivering water to a consumer (15) from a distribution pipe (16) which receives water from a transfer pipe (14) supplying water from a reservoir (12), said installation comprising:
• a probe (32) for measuring at least one parameter of the water flowing in the connection pipe (18);
• a contamination detector device (46) for detecting the presence, if any, of contamination on the basis of measurements performed by the probe;
• a reverse flow detector device (36) for detecting reverse flow in said connection pipe (18);
• a disinfection device (40) for disinfecting the water in the connection pipe (18);
• a cutoff valve (38) for closing the connection pipe (18); and
• a control member (50) for actuating the disinfection device (40) and/or the cutoff valve (38) as a function of data provided by the contamination detector device (46) and the reverse flow detector device (36), whereby the quality of the water delivered to or returned by the consumer (15) is monitored,
the entire installation being configured to be fitted to said connector pipe (18) connecting the consumer to the remainder of the network.

2. An installation according to claim 1, wherein it further includes a consumption meter (34) comprising said reverse flow detector device (36).

3. An installation according to claim 1 or 2, wherein it further includes a warning device for warning the consumer (15) and intended to be activated as a function of data supplied by the contamination detector device (46) and by the reverse flow detector device (36).

4. An installation according to any one of claims 1 to 3, wherein it further includes a transmission unit (49) for transmitting, to a control center (60) of a distribution network(10), information picked up by the contamination detector device (46) and the reverse flow detector device (36), together with the state of the disinfection device (40) and/or the state of the cutoff valve (38).

5. An installation according to any one of claims 1 to 4, wherein said parameter is taken at least from: chlorine concentration, conductivity, temperature, turbidity, and organic matter concentration.

6. An installation according to any one of claims 1 to 5, wherein the disinfection device (40) comprises an electrochemical system (42) generating oxidizing compounds.

7. An installation according to any one of claims 1 to 6, wherein the disinfection device comprises an ultraviolet lamp (44).

8. An installation according to any one of claims 1 to 7, wherein the probe measures at least two parameters, and wherein the contamination detector device (46) comprises a device for performing correlated analysis of said at least two parameters.

9. An installation according to any one of claims 1 to 8, wherein the probe (32) performs measurements without using any reagent and without rejecting water to the outside of the connection pipe (18).

10. Connection pipe, dedicated to delivering water to a consumer (15) from a distribution pipe (16) connected to a transfer pipe (14) supplying water from a reservoir (12), on which an installation (30) according to any one of claims 1 to 9 is mounted.

11. A distribution network (10) including a plurality of distribution pipes (16) connected to at least a transfer pipe (14) supplying water from a reservoir (12), a plurality of connection pipes (18) connected to the distribution pipes (16) for distributing water to consumers, and at least one installation (30) according to any one of claims 1 to 9, said at least one installation being entirely mounted on one of said connection pipes (18).

12. A distribution network according to claim 11, wherein it further includes a control center (60) receiving the information supplied by the contamination detector device (46) and the reverse flow detector device (36), together with the state of the disinfection device and/or the state of the cutoff valve, and wherein the control center also includes means for remotely controlling the control member of the installation.

13. A distribution network according to claim 12, wherein the control center (60) further includes a hydraulic model and/or a quality model receiving the data supplied by the contamination detector device (46) in such a manner as to predict the propagation in the network of detected contamination, if any.

14. A method of controlling the quality of water in a distribution network that includes at least one installation according to any one of claims 1 to 9, in which method the disinfection device (40) and/or the cutoff valve (38) is actuated when a reverse flow is detected and/or when contamination is detected.

15. A method of controlling water quality according to claim 14, wherein a warning is triggered if the disinfection device (40) and/or the cutoff valve (38) is/are actuated.
